# EUROPEAN PATENT SPECIFICATION

(11) **EP 2 106 862 B1**
(45) Date of publication and mention of the grant of the patent: **02.12.2015**
(21) Application number: 08160842.4
(22) Date of filing: 21.07.2008
(51) Int. Cl.: B06B 1/06, G10K 11/32, A61N 7/02, A61B 17/22

(54) **Procedure for the manufacturing of a piezoelectric ceramics radiating surface**
Verfahren zur Herstellung einer Strahlungsoberfläche aus piezoelektrischer Keramik
Procédé de fabrication de surface radiante en céramique piézoélectrique

(30) Priority: 02.04.2008 ES 200801025
(43) Date of publication of application: 07.10.2009
(73) Proprietor: Indiba, S.A., 08192 Sant Quirze del Valles (Barcelona) (ES)
(72) Inventor: Sanchez Soriano, Manuel, 08192, SANT QUIRZE DEL VALLÈS (ES); Amat Genis, Alberto, 08192, SANT QUIRZE DEL VALLÈS (ES); Bruguera Gudayol, Eduard, 08192, SANT QUIRZE DEL VALLÈS (ES); Vadillo Pastor, Juan Ramón, 08192, SANT QUIRZE DEL VALLÈS (ES); Corral Baqués, Marc Ignasi, 08192, SANT QUIRZE DEL VALLÈS (ES); Coussios, Constantin Cassios, Oxford, OX1 3PJ (GB); Kyriakou, Zoe, Oxford, OX1 3PJ (GR)
(74) Representative: Manresa Val, Manuel

(56) References cited:
- EP-A- 1 345 657
- EP-A- 1 774 989
- WO-A-2006/021651
- US-A1- 2008 027 328
- US-B1- 6 656 136

## Description

A procedure for the manufacturing of a piezoelectric ceramics radiating surface is provided, of the type that is used in a powerful ultrasound transducer, said transducer being next to an electrical impedances adaptation network, a power amplifier, a signal generator, a control unit, with the transducer also comprising an external casing, said piezoelectric ceramics between some electrodes, an absorbent material between the casing and the piezoelectric ceramics and an adaptation layer.

### BACKGROUND OF THE INVENTION

The applicant is known for its continuous innovations in the electro aesthetics and electro medicine sector.

European Patent No. 1345657, "Ultrasound lipolysis", from the year 2001, in the name of the Israeli firm INSIGHTEC-IMAGE GUIDED TREATMENT LTD. In this invention the cells are destroyed inside an area of subcutaneous tissue by ultrasound radiation produced by a transducer that is external and adjacent to the patient. The transducer emits the acoustic energy that is focused in a linear focus area inside the area of tissue. The acoustic energy is intense enough to produce the lysis of the cells inside the focus area reducing heating to a minimum. The transducer can be made up of one or several independent radiating elements that have a partially cylindrical shape, a single flat transducer element attached to an acoustic lens, or a plurality of linear transducer elements placed alongside each other in an arched or flat arrangement. The transducer can include detectors for detecting the cavitations that are produced in the focus area, which is correlated to the grade of cell destruction.

Previously, this type of semi cylindrical ultrasound emitting transducers have been, and are being, used for treating cancers that are localised and can be accessed by this technique, as can be appreciated in European Patent No. 20060255209 (published as EP 1 774 989) in the name of Larson, Eugene A. (2457 West Shore Drive, Lummi Island Washington 98262, US) Kaminski, Perry W. (1 Lake Shore Trail, Stehekin Washington 98852, US), which shows that the application of this type of ultrasound energy focusing elements precedes the above-mentioned European Patent No. 1345657.

US-B1-6656136 discloses an aspheric ultrasonic transducer.

### BRIEF DESCRIPTION OF THE INVENTION

This invention is an improvement in the sector that applies focused ultrasounds to the fields of medicine and aesthetics, in particular to high intensity focused ultrasound systems (also known as HIFU).

According to the inventors, the previous patents are limited in that the ultrasound energy is focused in an area that is as long as the emission surface, and in that an axis parallel to the cylinder, has defined energy focusing values that are smaller than those of a hemispherical transducer.

The inventors have developed a new procedure for obtaining or manufacturing a transducer that enables radiating a smaller focus volume than that of a semicylinder and greater than that of a hemisphere, thereby obtaining a greater energy focus than in a semicylinder and a greater focusing area than a hemisphere.

With reference to the semicylinder, the new radiating surface required a smaller amplifying capacity with the consequent saving in the design and manufacturing of the treatment equipment, which also meant that the resulting equipment worked more efficiently.

As a consequence of the design of this new surface, it is possible, by varying the different geometrical elements that form it, to vary the focal depth and area. This means that new treatment approaches can be performed, and also that the effective energies can be concentrated sufficiently in the focus area so as not to damage the adjacent structures or tissues that need not be treated, such as for example the skin.

This new procedure developed by the inventors, makes it possible to obtain a transducer which, depending on the depth and the volume of the focus area, can perform various treatments, including, for example, 15mm from the skin the fat area, 3-4 cm from the skin the treatment for the pain in the tendon or muscular tissue, and 2-3 mm from the skin it is possible to heat the dermal tissue to induce the cells to produce new collagen and elastic fibres; it is possible to treat the cancer, according to its location, selecting the focus and depth suitable to the type and location of the tumour in question.

The object of this invention is a procedure for the manufacturing of a piezoelectric ceramics radiating surface, of the type that is used in a powerful ultrasound transducer, said transducer being next to an electrical impedance adaptation network, a powerful amplifier, a signal generator, a control unit, with the transducer also comprising an external casing, said piezoelectric ceramics between some electrodes, an absorbent material between the casing and the piezoelectric ceramics and an adaptation layer, as claimed in claim 1 and a piezoelectric ceramics as claimed in claim 3. A preferred embodiment is disclosed in the dependent claim.

### BRIEF DESCRIPTION OF THE DRAWINGS

In order to facilitate the explanation twelve sheets of drawings accompany this specification, which illustrate a practical embodiment, and which is provided as a non-limiting example of the scope of this invention:
- Figure 1 is a block diagram showing a system for producing acoustic energy.
- Figures 2a to 2m are a sequence of drawings illustrating the various stages of the procedure.
- Figure 3a is a plane view of the ceramics.
- Figure 3b is a side view of the ceramics.
- Figure 3c is a front view of said ceramics.
- Figure 4 is a perspective view of a piezoelectric headpiece.
- Figure 5 is a section along the line V-V in Figure 4.
- Figure 6 is a side tomography of the ultrasound action under the skin, and
- Figure 7 is a plane tomography of the ultrasound action under the skin.

### SPECIFIC EMBODIMENT OF THIS INVENTION

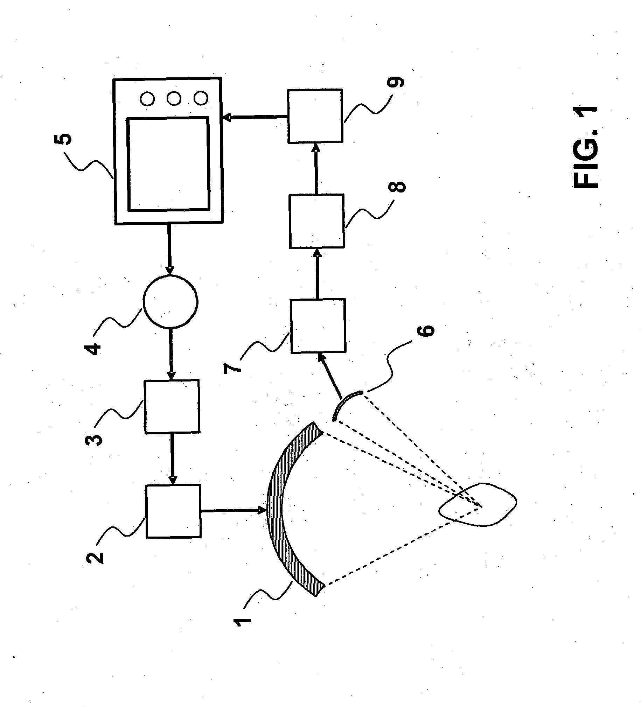

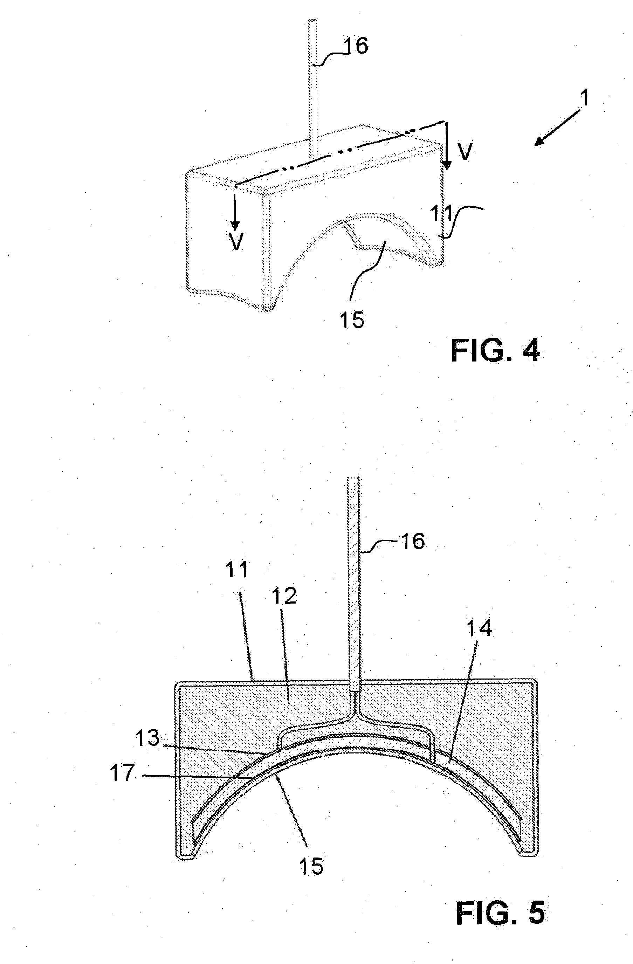

So, in a specific embodiment, a description is provided of the procedure for the manufacturing of a piezoelectric ceramics radiating surface, of the type that is used in a powerful ultrasound transducer 1 (Figs. 1 and 4).

Said transducer 1 is part of a system capable of producing the desired acoustic energy in a controlled fashion. (Fig. 1)

The piezoelectric ceramics radiating surface U2 is defined as part of a surface U1 that contains an arch A2 and is limited by two projections p1 and p2, without necessarily containing them.

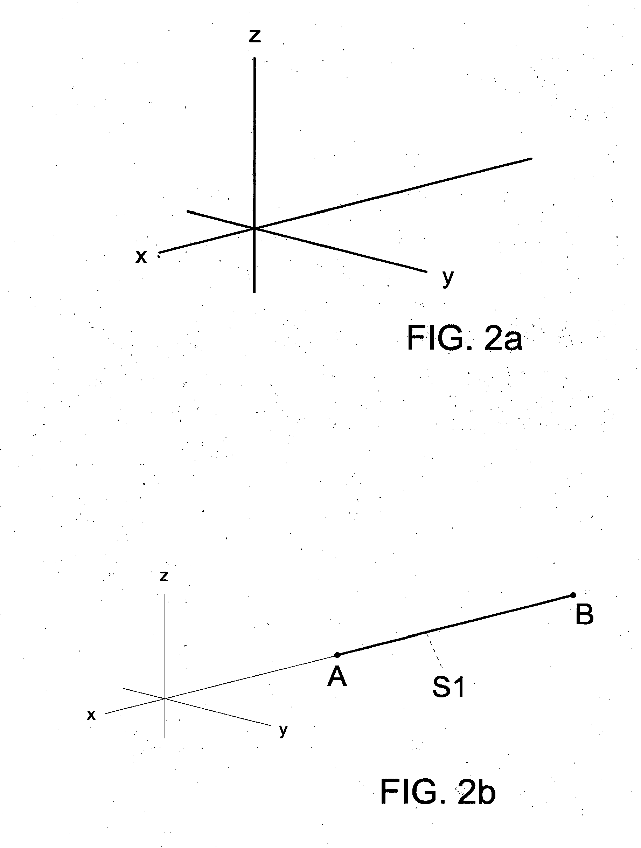

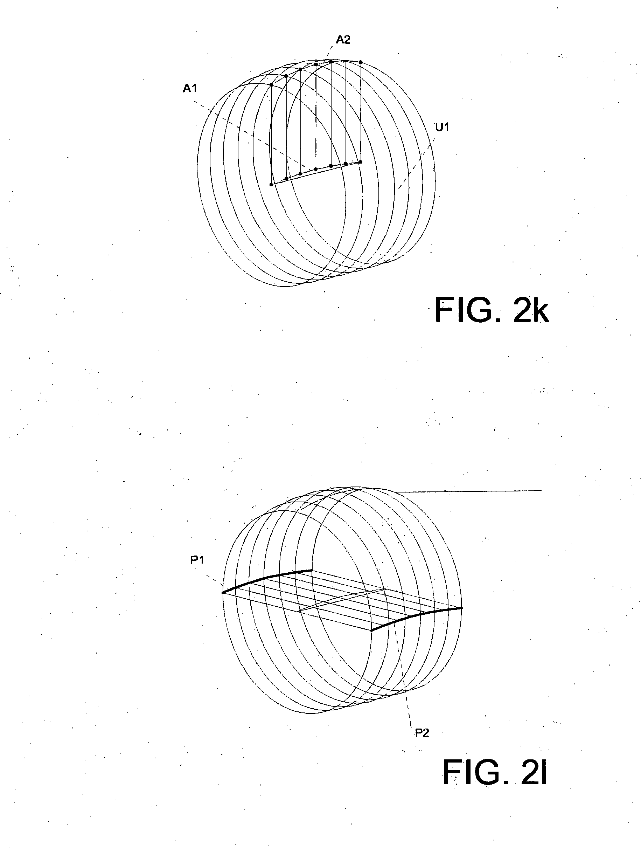

This surface is obtained by applying the procedure set out below. First of all the three space axes X, Y and Z are determined (Fig. 2a).

Then a first segment S1 is drawn along axis X, which determines ends A and B (Fig. 2b).

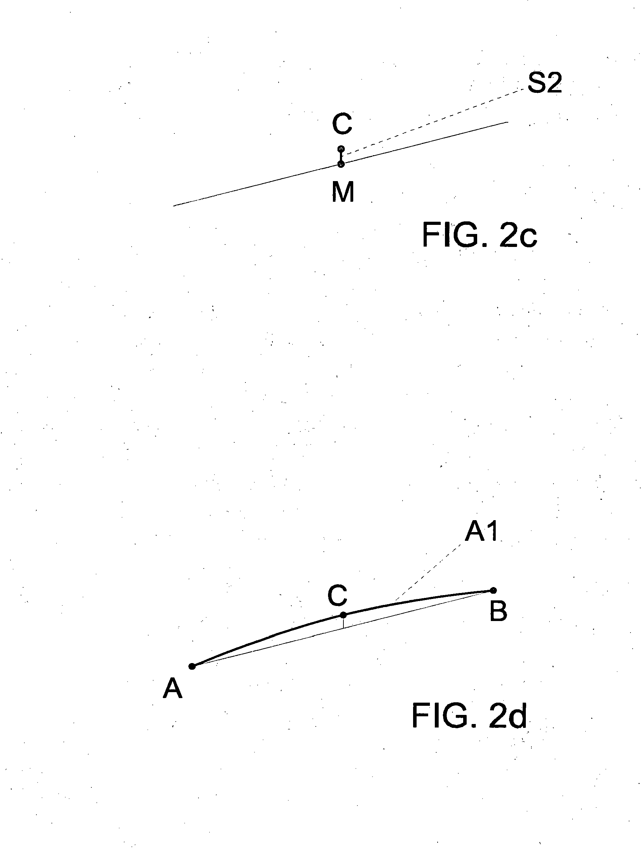

Then a second segment S2 is drawn, with a length h other than zero, originating in the medium point M of the first segment S1 and parallel to axis Z that ends in point C (Fig. 2c).

Then, a first arch A1 is defined that passes through points A, C and B, defined beforehand, corresponding with the ends of segment S1 and one of the ends of segment S2 (Fig. 2d).

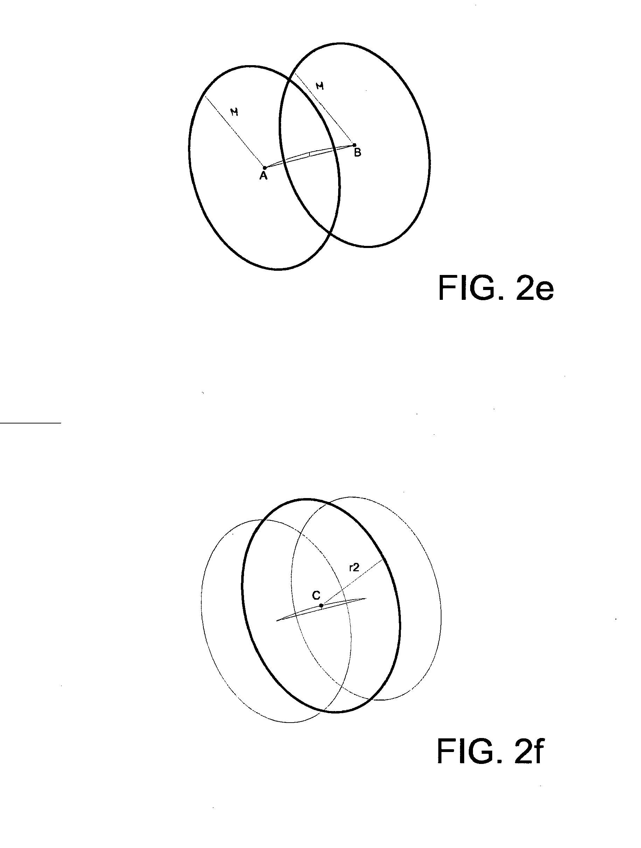

Then two circumferences are drawn with radius r1 centred in said points A and B. Said circumferences are parallel to plane YZ (Fig. 2e).

Next a circumference is drawn parallel to the previous ones with radius r2 and centred in point C, which is the end of segment S2 (Fig. 2f).

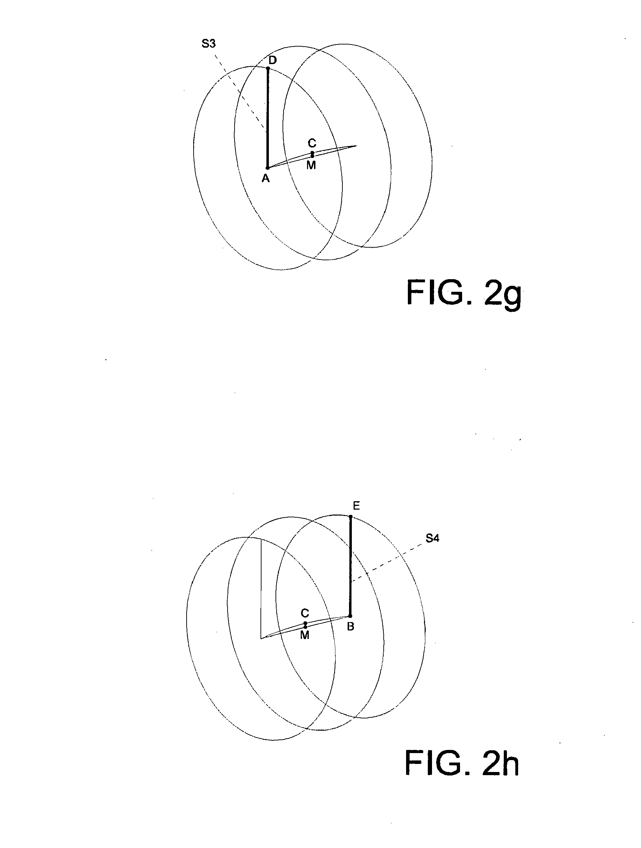

Afterwards a vector (vector MC) is defined starting in the point M of the first segment S1 and ending in point C, which as stated above, is the end of the second segment S2. A third segment S3 is drawn originating in point A of the first segment S1, in plane XZ, with length r1 in the direction of vector MC, with an end point of said segment being defined, point D (Fig. 2g).

Subsequently a fourth segment S4 is drawn originating in point B of the first segment S1, orientated in plane XZ, with length r1 in the direction of vector MC, with an end point of said fourth segment being defined, point E (Fig. 2h).

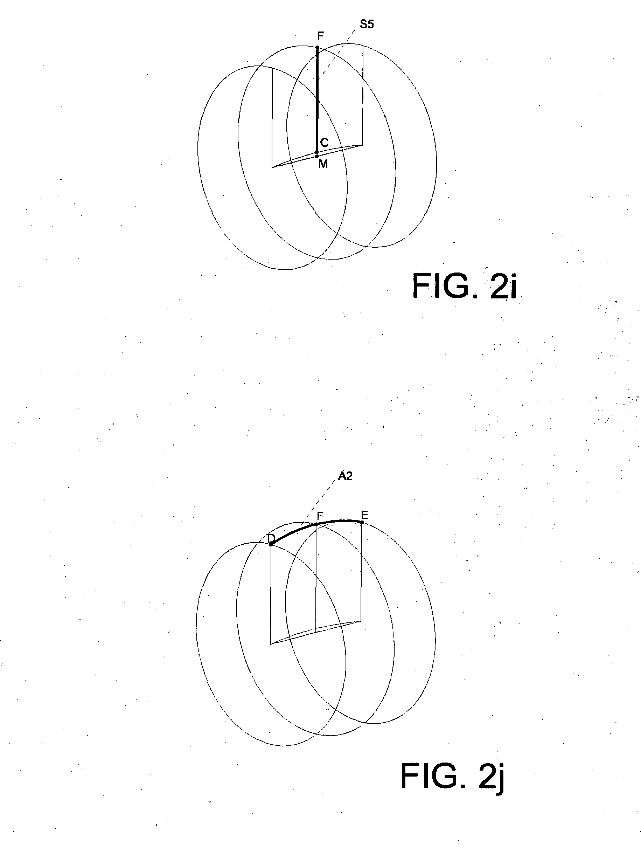

Then a fifth segment S5 is drawn originating in point C, which is the end of segment S2, in plane XZ, with length r2 in the direction of vector MC, with an end point of said fifth segment S5 being defined, point F (Fig. 2i).

Afterwards a second arch A2 is defined that passes through the above-mentioned points D, F and E (Fig. 2j).

Then a surface U1 is defined made up of all the circumferences parallel to plane YZ, centred in the first arch A1 and with one point of the perimeter thereof located in the second arch A2 (Fig. 2k).

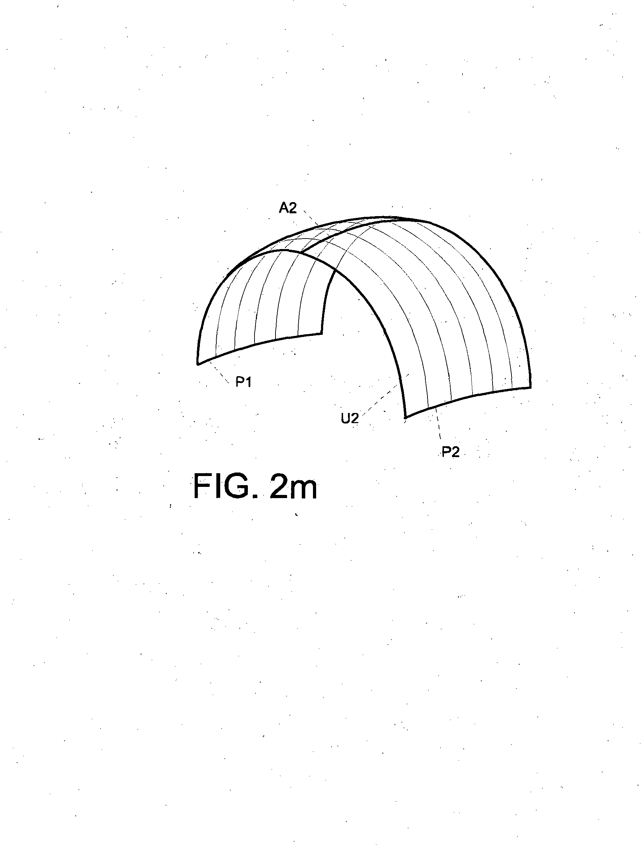

Finally the first arch A1 is projected in the direction of axis Y on surface U1 producing two projections p1 and p2 which, when joined together, form piezoelectric ceramics radiating surface U2 (Fig. 2I), which can be cut as required (Fig. 2m).

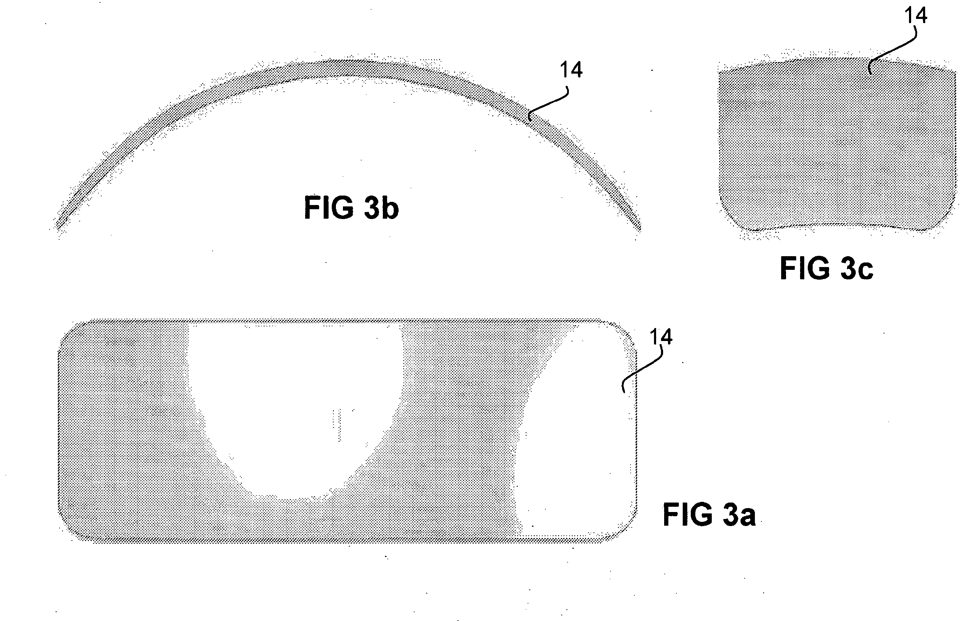

One of the embodiments of said piezoelectric ceramics in Figure 2m, is like the one shown in Figures 3a, 3b and 3c, which are a plane, side and front view of that shown in said Figure 2m.

According to requirements, radius r1 can be different from r2 or radii r1 and r2 can be identical.

The said system, in Figure 1, comprises a control unit 5 that manages the emission time and the amplitude of the signal supplied to the amplifier. Also, this unit will set up other operating parameters, such as the emission mode, which can be continuous or pulsed, the working cycle, the number of impulses, etc.

Signal generator 4 supplies the power amplifier 3 with an essential emission frequency, which is adjustable and coincides with the transducer resonance frequency. This generator can, possibly, work with harmonics in the essential frequency, in order to vary the transducer emission wavelength, and this way, reduce the size of the focus area.

The electrical impedance adaptation network 2 adapts the signal that leaves the amplifier 3 so that transducer 1 receives it correctly.

In one embodiment it can be foreseen that the system detects whether the acoustic energy is deposited in the transducer focus. To this end, a detection transducer 6 is added to the above-mentioned system, focused in the focus area of power transducer 1, to know whether cavitations occur in the focus point; a signal amplifier 8 is also added, if the received signal is weak; and a signal treatment block 9, which would be connected to control unit 5, which analyses the signal to detect the cavitations; and an electrical impedance adaptation network 7 which adapts the signal that leaves transducer 6 so that it is received correctly by amplifier 8.

Figure 4 shows transducer 1 with its casing 11, an electrical lead 16 and an adaptation layer 15, which is explained in further detail in Figure 5.

Figure 5 is the section along the line V-V in Figure 4, which illustrates casing 11, lead 16, an absorbent material 12, a piezoelectric ceramics 14 sandwiched by a first metallic electrode 13 and a second metallic electrode 17 and adaptation layer 15.

This way, lead 16 with two connectors is attached to electrical impedances adaptation network 2.

Casing 11 surrounds and protects the various elements making up transducer 1.

Absorbent material 12 absorbs the vibrations of piezoelectric ceramics 14 inside transducer 1.

First metallic electrode 13 covers the whole top surface of the piezoelectric ceramics, to which one of the connectors of lead 16 is connected, and the second metallic electrode 17 covers the whole bottom surface of piezoelectric ceramics 14, to which the other connector of electric lead 16 is connected.

Piezoelectric ceramics 14 produces the ultrasound vibration when it is excited to a certain frequency by the two metallic electrodes 13,17. Said vibration is transmitted to the adaptation layer 15 which adapts the acoustic impedances of transducer 1 and of the medium (the part of the human body) that is going to be the exposed to the acoustic field.

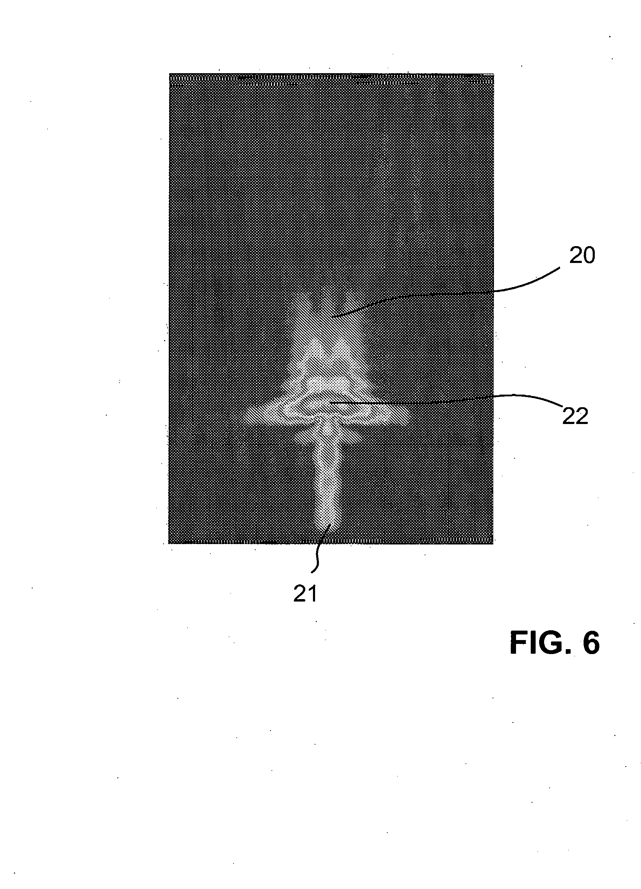

Figure 6 is a side tomography of the ultrasound action under the skin where a prefocus area 20, post focus area 21 and an area 22 can be seen, the latter being where the acoustic pressure is greater and therefore where the temperature is also higher.

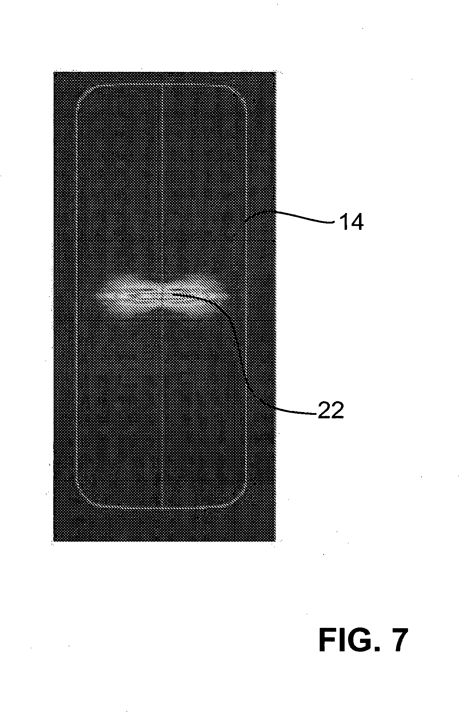

Figure 7 is a plane tomography of the ultrasound action under the skin, after creating a transparent effect on piezoelectric ceramics 21 to make it easier to see the tomography.

This invention describes a new procedure for the manufacturing of a piezoelectric ceramics radiating surface. The examples mentioned herein do not limit the invention, and therefore it can have different applications and/or adaptations, all within the scope of the following claims.

## Claims

1. Procedure for the manufacturing of a piezoelectric ceramics radiating surface, of the type that is used in a powerful ultrasound transducer, said transducer being next to an electrical impedance adaptation network, a power amplifier, a signal generator, a control unit, said transducer also comprising an external casing, said piezoelectric ceramics between some electrodes, an absorbent material between the casing and piezoelectric ceramics and an adaptation layer, wherein the piezoelectric ceramics radiating surface U2 is obtained by applying the following procedure:
- the three space axes X, Y and Z are determined
- a first segment S1 is drawn along axis X between two points A and B,
- a second segment S2 parallel to axis Z is drawn, with a length h other than zero, between a middle point M of the first segment S1 and a point C,
- a first arch A1 is defined that passes through points A, C and B,
- two circumferences are drawn with radius r1 centred in points A and B and in planes parallel to plane YZ,
- a circumference is drawn parallel to the previous ones with radius r2, centred in C;
- a vector is defined that starts at point M and ends at point C,
- a third segment S3 in plane XZ, in the direction of vector MC, is drawn between the point A and point D, being defined by length r1,
- a fourth segment S4 in plane XZ, in the direction of vector MC, is drawn between the point A and point E, being defined by length r1
- a fifth segment S5 is in plane XZ, in the direction of vector MC, is drawn between the point C and point F, being defined by length r2,
- a second arch A2 is defined that passes through points D, F and E,
- a surface U1 is defined made up of all the circumferences parallel to plane YZ centred in the first arch A1 and with one point of the perimeter thereof located in second arch A2, and
- first arch A1 is projected in the direction of axis Y on surface U1 producing two projections p1 and p2,
- finally surface U2 is cut as required,
such that said ceramics radiating surface U2 is defined as part of a surface U1 that contains an arch A2 and is limited by two projections p1 and p2, without necessarily containing them.

2. Procedure according to claim 1 **characterized in that** radius r1 is different to radius r2.

3. Piezoelectric ceramics manufactured according to the procedure of claim 1 **characterised in that** it adopts a form of double arch, transversally and longitudinally curved.

## Patentansprüche

1. Verfahren zur Herstellung einer Abstrahlfläche aus piezoelektrischer Keramik der Art, wie sie in einem leistungsstarken Ultraschall-Messumformer verwendet wird, wobei sich dieser Messumformer neben einem elektrischen Widerstandsanpassungsnetz, einem Leistungsverstärker, einem Signalgenerator, einem Steuergerät befindet, und dieser Messumformer auch ein externes Gehäuse, die piezoelektrische Keramik zwischen einigen Elektroden, ein absorbierendes Material zwischen dem Gehäuse und der piezoelektrischen Keramik und eine Adapterschicht umfasst, wobei die Abstrahlfläche aus piezoelektrischer Keramik U2 durch Anwendung des folgenden Verfahrens erhalten wird:
- die drei räumlichen Achsen X, Y und Z werden bestimmt
- ein erstes Segment S1 wird entlang der X-Achse zwischen den zwei Punkten A und B gezogen,
- ein zweites Segment S2 parallel zur Z-Achse wird gezogen mit einer Länge h größer als null zwischen einem Mittelpunkt M des ersten Segments S1 und einem Punkt C,
- ein erster Bogen A1 wird definiert, der durch die Punkte A, C und B verläuft,
- zwei Kreisumfänge mit Radius r1 werden zentriert in den punkten A und B und auf parallelen Ebenen zur Ebene YZ gezogen,
- ein Kreisumfang mit Radius r2 und zentriert in C wird parallel zu den vorherigen gezogen,
- ein Vektor wird definiert, der am Punkt M beginnt und am Punkt C endet,
- ein drittes Segment S3 auf der Ebene XZ in der Richtung des Vektors MC wird gezogen zwischen dem Punkt A und Punkt D, definiert durch die Länge r1,
- ein viertes Segment S4 auf der Ebene XZ in der Richtung des Vektors MC wird gezogen zwischen dem Punkt A und Punkt E, definiert durch die Länge r1,
- ein fünftes Segment S5 auf der Ebene XZ in der Richtung des Vektors MC wird gezogen zwischen dem Punkt C und Punkt F, definiert durch die Länge r2,
- ein zweiter Bogen A2 wird definiert, der durch die Punkte D, F und E verläuft,
- eine Fläche U1 wird definiert, die aus allen Kreisumfängen parallel zur Ebene YZ besteht, zentriert im ersten Bogen A1 und mit einem Punkt von dessen Umfang auf dem zweiten Bogen A2, und
- der erste Bogen A1 wird in die Richtung der Y-Achse auf der Fläche U1 projektiert und erzeugt zwei Überstände p1 und p2,
- abschließend wird die Fläche U2 wie erforderlich zugeschnitten,
- derart, dass die keramische Abstrahlfläche U2 als Teil einer Fläche U1 definiert ist, die einen Bogen A2 enthält und durch zwei Überstände p1 und p2 begrenzt wird, ohne dass diese zwingend enthalten sein müssen.

2. Verfahren gemäß Anspruch 1, **dadurch gekennzeichnet, dass** der Radius r1 sich vom Radius r2 unterscheidet.

3. Piezoelektrische Keramik hergestellt gemäß dem Verfahren von Anspruch 1, **dadurch gekennzeichnet, dass** sie die Form eines doppelten, transversal und longitudinal gebogenen Bogens annimmt.

## Revendications

1. Procédure de fabrication de surface radiante en céramique piézoélectrique, du type qui est utilisé dans un puissant transducteur ultrasonique, ledit transducteur étant à côté d'un réseau d'adaptation d'impédance électrique, un amplificateur de puissance, un générateur de signaux, une unité de contrôle, ledit transducteur comprenant également un boîtier extérieur, ladite céramique piézoélectrique étant entre des électrodes, un matériel absorbant entre le boîtier et la céramique piézoélectrique et une couche d'adaptation, où la surface radiante en céramique piézoélectrique U2 est obtenue en appliquant la procédure suivante :
- les trois axes d'espace X, Y et Z sont déterminés
- un premier segment S1 est tracé le long d'un axe X entre deux points A et B,
- un second segment S2 est tracé parallèle à l'axe Z, d'une longueur h autre que zéro, entre un point situé au milieu M du premier segment S1 et un point C,
- un premier arc A1 est défini qui passe par les points A, C et B,
- deux circonférences sont tracées d'un rayon r1 centrées aux points A et B et sur des plans parallèles au plan YZ,
- une circonférence est tracée parallèlement aux précédentes avec un rayon r2, centré en C,
- un vecteur est défini qui part du point M et qui termine au point C,
- un troisième segment S3 sur le plan XZ, dans la direction du vecteur MC, est tracé entre le point A et le point D, étant défini par la longueur r1,
- un quatrième segment S4 sur le plan XZ, dans la direction du vecteur MC, est tracé entre le point A et le point E, étant défini par la longueur r1,
- un cinquième segment S5 sur un plan XZ, dans la direction du vecteur MC, est tracé entre le point C et le point F, étant défini par la longueur r2.
- un deuxième arc A2 est défini qui passe par les points D, F et E.
- une surface U1 est définie, faite de toutes les circonférences parallèles au plan YZ centrée sur le premier arc A1 et dont un point du périmètre est situé sur le deuxième arc A2, et
- un premier arc A1 est projeté dans la direction de l'axe Y sur la surface U1 produisant deux projections p1 et p2,
- finalement, une surface U2 est coupée comme requis,
de telle manière que la surface radiante en céramique U2 soit définie comme une partie d'une surface U1 contenant un arc A2 et soit limitée par deux projections p1 et p2 sans nécessairement les contenir.

2. Procédure conformément à la revendication 1, **caractérisée en ce que** le rayon r1 est différent du rayon r2.

3. Céramique piézoélectrique fabriquée conformément à la procédure de la revendication 1, **caractérisée en ce qu'**elle adopte une forme d'arc double, cintré transversalement et longitudinalement.
